Europäisches Patentamt

European Patent Office

Office européen des brevets

(11) Veröffentlichungsnummer: **0 414 032 A1**

# EUROPÄISCHE PATENTANMELDUNG

(21) Anmeldenummer: 90115117.5

(51) Int. Cl.5: **G01N 27/28**

(22) Anmeldetag: 07.08.90

(30) Priorität: 18.08.89 DE 8909902 U

(43) Veröffentlichungstag der Anmeldung:
27.02.91 Patentblatt 91/09

(84) Benannte Vertragsstaaten:
AT BE CH DE DK ES FR GB GR IT LI LU NL SE

(71) Anmelder: Conducta Gesellschaft für Mess-
und Regeltechnik mbH & Co.
Dieselstrasse 24

D-7016 Gerlingen b. Stuttgart(DE)

(72) Erfinder: Stellmacher, Klaus
Poststrasse 22
D-7261 Oberreichenbach(DE)

(74) Vertreter: Otte, Peter, Dipl.-Ing.
Tiroler Strasse 15
D-7250 Leonberg(DE)

(54) Elektrodenhalterung für Eintauch-, Durchfluss- und Anbaumesssysteme in der analytischen Chemie.

(57) Bei einer Elektrodenhalterung für Eintauch-, Durchfluß-und Anbau-Meßsysteme in der analytischen Chemie, insbesondere für die pH-, Redox-, Leitfähigkeits-, Sauerstoff- und Chlormessung u.dgl., mit einem mindestens eine Elektrode abgedichtet aufnehmenden Lagerrohr für diese, welches in das Meßmedium eintaucht, wird vorgeschlagen, zwischen dem die Elektrode aufnehmenden inneren Lagerrohr (11) und einem äußeren, das Lagerrohr bis in den Bereich des Meßmediums umgebenden und über den Spiegel des Meßmediums um einen vorgegebenen Abstand hinausragenden Schutz- oder Halterohr (12) eine Bajonettverschlußverbindung (14) vorzusehen, wobei sich oberhalb und unterhalb der Bajonettverschlußverbindung (14) jeweils mindestens eine Dichtung (17, 18) befindet.

**Fig.1**

## ELEKTRODENHALTERUNG FÜR EINTAUCH-, DURCHFLUSS- UND ANBAU-MESSSYSTEME IN DER ANALY-TISCHEN CHEMIE

Stand der Technik

Die Erfindung geht aus von einer Halterung für Elektroden in der analytischen Chemie nach dem Oberbegriff des Anspruchs 1.

Elektrodenhalterungen, die so ausgebildet sind, daß sie eine geeignete Meßelektrode, beispielsweise pH-Elektrode, mit einem Meßmedium etwa in Form einer Durchfluß-, Anbauarmatur oder Eintaucharmatur in Verbindung bringen, sind in vielfältiger Form bekannt.

Da die Meßmedien nicht selten stark elektrodenbelastende, etwa abrasive Eigenschaften haben und darüber hinaus beispielsweise bei Gehalt an Kalziumcarbonat mit Ablagerungen an den Armaturen reagieren, ist es erforderlich, die Elektrode zu gegebenen Zeitpunkten, nicht zuletzt auch zu Eichzwecken, aus der Halterung herauszunehmen, wobei dann auch eine Reinigung bezüglich der Ablagerungen vorgenommen werden kann. Dabei wird üblicherweise so vorgegangen, daß beim Ausbau der Meßarmatur aus dem Behälter mit dem Meßmedium, in welchem sie sich befindet, die Elektrode aus ihrem Halter entnommen wird, in den sie beispielsweise von oben oder von unten eingesetzt ist. Dabei kann die Elektrode mit dem Halter dadurch verbunden sein, daß sie mittels einer Überwurfmutter mit Innen-oder Außengewinde in dem Halter abgedichtet verschraubt ist, wobei Überwurfmutter und die Elektrodenhalterung üblicherweise aus einem geeigneten, entsprechend widerstandsfähigen Kunststoffmaterial bestehen. Problematisch ist in diesem Zusammenhang aber der Umstand, daß gerade bei abrasiven oder mit Ablagerungen reagierenden Medien, allgemein also überall dort, wo auch mit sandartigen Erscheinungsformen im Meßmedium gerechnet werden muß, diese Sandkörner natürlich auch in die Gewindegänge im Halterungsbereich gelangen können, was aber mit anderen Worten bedeutet, daß das Gewinde "frißt" und mindestens zur Schwergängigkeit neigt bzw. festgeht. Es kommt dann zu bestimmten Kaltverschweißungserscheinungen, die weiter dazu führen können, daß die jeweilige Bedienungsperson meint, das Gewinde schon auf Anschlag, also in den abgedichteten Bereich der gesamten Halterung gedreht zu haben, wenn dies tatsächlich noch nicht der Fall ist, so daß beim Einsetzen einer solchermaßen unzugänglich abgedichteten Durchfluß- oder Eintaucharmatur auch die inneren Anschlußbereiche und der rückwärtige Elektrodenbereich mit dem Meßmedium in Kontakt geraten und die ganze Anlage dann schnell unbrauchbar wird, da sie im untergetauchten Zustand an allen Stellen mit dem Meßmedium volläuft.

Der Erfindung liegt daher die Aufgabe zugrunde, bei einer Elektrodenhalterung in der analytischen Chemie, die allgemein für die Verwendung von Elektroden bei Eintauch-, Anbau- oder Durchfluß-Meßsystemen eingesetzt werden kann, dafür zu sorgen, daß auch bei stark abrasiven Medien die Dichtigkeit nicht nur im Elektrodenbereich, sondern auch mit Bezug auf den Befestigungsbereich frei von störenden Einflüssen, die aus dem Meßmedium herrühren, gehalten werden kann.

Vorteile der Erfindung

Die Erfindung löst diese Aufgabe mit den kennzeichnenden Merkmalen des Hauptanspruchs und hat den Vorteil, daß durch die beidseitige Dichtung mit Bezug auf den Befestigungsbereich sichergestellt ist, daß sowohl der Befestigungsbereich selbst, der im vorliegenden Fall durch einen Bajonettverschluß realisiert ist, als auch weiterführend der sich an den Befestigungsbereich anschließende Meßelektrodenbereich mit erhöhter Sicherheit gegen das Eindringen des Meßmediums geschützt ist, wobei sich noch der zusätzliche Vorteil ergibt, daß durch die Ausbildung des Befestigungsbereichs als Bajonettverschluß unter Verzicht auf eine Vielzahl von Gewindegängen auch weniger geschickten Bedienungspersonen verständlich wird, daß dann, wenn man nicht mindestens den Bajonettverschluß bis zum Einschnappen gedreht hat, der dichte Einsatz des Elektrodenhalterungs-Innenrohrs in das weiterführende Außenrohr noch nicht gelungen ist. Mit anderen Worten, es läßt sich wesentlich leichter feststellen, ob ein Bajonettverschluß in seine Endposition überführt worden ist, als beispielsweise mitzuzählen, wie viele Gewindegänge man beim Anschrauben einer Überwurfmutter in der Zwischenzeit zurückgelegt hat, bis auch die Dichtung auf Anschlag gebracht ist.

Von weiterem Vorteil ist die besonders einfache Ausbildung im gesamten Halterungsbereich für die Elektrode, wobei dennoch ein sicherer und abgedichteter Sitz erreicht werden kann, der darüber hinaus auch Anforderungen einer Sterilapplikation entspricht.

Durch die in den Unteransprüchen aufgeführten Maßnahmen sind vorteilhafte Weiterbildungen und Verbesserungen der im Hauptanspruch angegebenen Elektrodenhalterung möglich. Dabei können je nach Anzahl der von der Elektrodenhalterung aufzunehmenden Elektroden über den Umfang mehrere Teilgewindegänge der Bajonett- oder Verschlußsysteme vorgesehen sein.

Zeichnung

Ein Ausführungsbeispiel der Erfindung ist in
der Zeichnung dargestellt und wird in der nachfolgenden Beschreibung näher erläutert. Es zeigen:
Fig. 1 in einer teilweisen Längsschnittdarstellung
eine Elektrodenhalterung mit innerem Lagerrohr
für die Meßelektrode und äußerem Schutzrohr
und
Fig. 2 einen Querschnitt längs der Linie II-II.

Beschreibung der Ausführungsbeispiele

Der Grundgedanke vorliegender Erfindung liegt
darin, daß eine doppelte Abdichtung zwischen einem inneren, die Elektrode unmittelbar aufnehmenden Lagerrohr und einem äußeren Verschlußrohr
vorgesehen ist, die so ausgebildet ist, daß eine
erste Abdichtung für die Bajonettverschlußeinrichtung und eine zweite Abdichtung auf der anderen
Seite der Bajonettverschlußeinrichtung vorgesehen
ist, die beide z.B. als O-Ringe ausgebildet sind und
so dafür sorgen, daß mit Bezug auf das Meßsystem selbst eine doppelte Abdichtung vorliegt, wobei auch der Bajonettverschluß noch gegen die
Wirkung abrasiver Medien sicher geschützt ist.

Selbst wenn also die Abdichtung zum Bajonettverschluß ganz oder teilweise undicht zu werden
droht oder undicht wird, ist durch die zweite Abdichtung sichergestellt, daß das Elektrodensystem
mit Kontaktsteckern, elektrischen Anschlüssen
u.dgl. nicht mit dem Meßmedium in Kontakt
kommt, natürlich bis auf den sensitiven Elektrodenbereich, der so ausgebildet ist, daß sich dieser aus
dem inneren Lagerrohr zur freien Zugänglichkeit
durch das Meßmedium erstreckt.

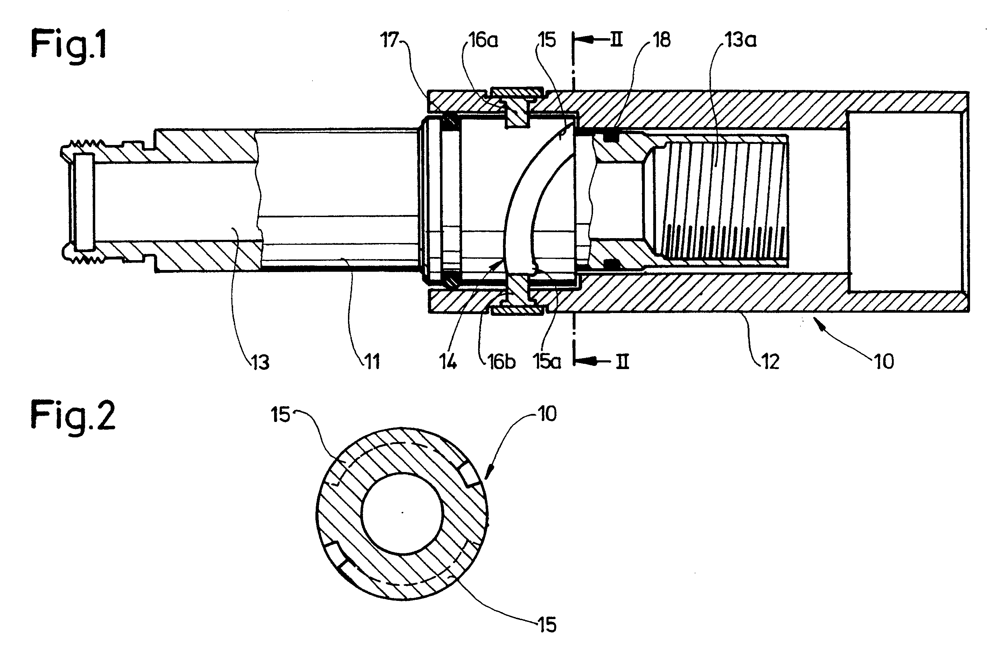

In Fig. 1 ist lediglich die Elektrodenhalterung
10 ohne die im inneren Lagerrohr 11 gehaltene
Elektrode dargestellt; dabei befindet sich das innere Lagerrohr 11 mit der von ihm aufgenommenen
Elektrode üblicherweise mindestens teilweise oder
vollständig untergetaucht unterhalb des Spiegels
des Meßmediums und lediglich ein zweites Überrohr oder Schutzrohr 12, welches mit dem Lagerrohr 11 in noch zu beschreibender Weise abdichtend verbunden ist, ragt dann über den Spiegel
des Meßmediums hinaus, gegebenenfalls erheblich, so daß jedenfalls von oben, wie in der Zeichnung dargestellt, keine Flüssigkeit in die Elektrodenhalterung mehr eindringen kann. Daher kann
das äußere Schutzrohr den Erfordernissen entsprechend nach oben beliebig verlängert werden, so
daß die Elektrodenhalterung für Eintauch- oder Durchflußmeßsysteme besonders geeignet ist. Die
Elektrode befindet sich dann im inneren Hohlraum
13 des Lagerrohrs 11, wobei vorzugsweise beide
Rohre 11 und 12 zylindrisch ausgebildet sein können und das äußere Schutzrohr 12 mindestens
teilweise über das innere Lagerrohr 11 für die
Elektrode gestülpt ist und dieses konzentrisch über
einen vorgegebenen Abstand umgibt.

Im Bereich 13a des inneren Aufnahmehohlraums 13 des Lagerrohrs 11 kann sich dann beispielsweise das Oberteil, also der Kopf des Elektrodenmeßsystems, beispielsweise pH-Elektrode befinden, mit den Anschlüssen, Steckkontakten u.dgl.,
die in der Zeichenebene dann weiter nach oben
längs des äußeren Schutzrohrs 12 hinausgeführt
sind.

Die (nicht dargestellte) Elektrode kann dann in
das innere Lagerrohr 11 eingegeben werden, wenn
dieses aus dem äußeren Schutz- oder Halterohr 12
gelöst ist, wozu entsprechend vorliegender Erfindung die folgenden Mittel vorgesehen sind. Zwischen dem äußeren Schutzrohr oder Halterohr 12
und dem inneren Lagerrohr 11 besteht eine Bajonettverschlußverbindung 14, die, wie es sich versteht, in ihrer allgemeinen Ausführung für sich gesehen bei Verschlußsystemen bekannt ist; beispielsweise können bei vorliegender Erfindung an
gegenüberliegenden Seiten in die Außenwandung
des inneren Lagerrohrs 11 zunächst schräg nach
unten und dann für eine kurze Distanz in etwa
horizontal verlaufende Führungsschlitze 15 eingefräst oder sonstwie eingebracht sein; in diese Führungsschlitze greifen beidseitige Vorsprünge 16a,
16b, die im unteren Bereich des äußeren Schutzrohrs 12 nach innen ragend vorgesehen sind, so
ein, daß das äußere Schutzrohr bei der Montage
nach eingelegter Elektrode in das innere Lagerrohr
von oben auf das innere Lagerrohr 11 zunächst
konzentrisch und durch axiale Längsverschiebung
aufgeschoben wird, bis die Vorsprünge 16a, 16b in
die Führungsnuten 15 des Bajonettverschlusses
einfallen; anschließend werden die beiden Rohre
11 und 12 gegeneinander verdreht, so daß sie
durch die nunmehr automatische Führung des Bajonettverschlusses tiefer konzentrisch ineinandergleiten und gleichzeitig im Endbereich eine Verriegelung stattfindet, beispielsweise durch den horizontalen Verlauf an dieser Stelle oder indem eine
kleine nach oben gerichtete Ausnehmung 15a jedes Führungsschlitzes dafür sorgt, daß die Vorsprünge 16a, 16b an dieser Stelle in eine gewisse
Verrastungswirkung geraten. Um daher die beiden
Rohre voneinander wieder lösen zu können, muß
eine bewußte kräftige Verdrehung in Gegenrichtung
erfolgen, woraufhin dann das äußere Schutz- oder
Halterohr 12 sich allmählich immer stärker axial
nach oben bewegt und vom inneren Lagerrohr 11
löst.

Ein wesentliches weiteres Merkmal bei dieser Verriegelungsmöglichkeit zwischen den beiden Rohren besteht dann noch darin, daß sowohl unterhalb als auch oberhalb des Bajonettverschlusses 14 jeweils Dichtungen zwischen den beiden Rohren vorgesehen sind, und zwar z.B. ein unterer O-Ring 17 und ein oberer O-Ring 18. Man erkennt, daß diese O-Ringdichtungen 17 und 18 den Bereich des Bajonettverschlusses 14 zwischen sich einschließen und daher einmal dafür sorgen, daß die abrasiven Medien oder mit den Meßmedien mitgeführten Stoffe nicht von unten in den Gleit- und Arbeitsbereich des Bajonettverschlusses 14 gelangen können, wofür die Dichtung 17 sorgt, während die sich darüber befindende Dichtung 18 einen zusätzlichen Schutz des Elektrodensystems sicherstellt, da auch dann, wenn möglicherweise der Bereich des Bajonettverschlusses durch das Meßmedium überflutet ist, durch einen eventuellen

Dichtungsfehler, die Dichtung 18 weiter sicher dafür sorgt, daß jedenfalls das Elektrodenmeßsystem unbeeinflußt weiterarbeiten kann.

Da ein Versagen beider Dichtungen nahezu ausgeschlossen ist, sichert die vorliegende Erfindung in Verbindung mit dem Bajonettsystem und den beiden Dichtungen eine einwandfreie Funktion des Meßelektrodensystems als Eintauchsystem, Anbau- oder Durchflußsystem gerade bei stark belastenden Medien, wie diese sich beispielsweise etwa bei der Zinkaufbereitung oder bei Entschwefelungsanlagen ergeben, wo bisher nur mit extrem kurzen Lebensdauern (lediglich einige Wochen) der jeweils verwendeten Meßsysteme gerechnet werden konnte. Darüber hinaus ist noch von Bedeutung, daß mit der gefürchteten Reibungsverschweißung im Bereich des Bajonettverschlusses kaum gerechnet zu werden braucht, einmal natürlich wegen der ohnehin vorhandenen unteren Dichtung 17, aber auch deshalb, weil eine Bedienungsperson die kurzen Drehwege des Bajonettverschlusses sicher erfassen und auch durchführen wird, bis die Endarretierpositionen erreicht sind, während dies bei den bisherigen Techniken, die auf dem Festschrauben von Überwurfmuttern beruhen, selten der Fall ist und auch nicht erwartet werden kann.

**Ansprüche**

1. Elektrodenhalterung für Eintauch-, Durchfluß- und Anbau-Meßsysteme in der analytischen Chemie, insbesondere für die pH-, Redox-, Leitfähigkeits-, Sauerstoff- und Chlormessung u. dgl., mit einem mindestens eine Elektrode abgedichtet aufnehmenden Lagerrohr für diese, welches in das Meßmedium eintaucht, dadurch gekennzeichnet, daß zwischen dem die Elektrode aufnehmenden inneren Lagerrohr (11) und einem äußeren, das Lagerrohr bis in den Bereich des Meßmediums umgebenden und über den Spiegel des Meßmediums um einen vorgegebenen Abstand hinausragenden Schutz- oder Halterohr (12) eine Bajonettverschlußverbindung (14) vorgesehen ist, wobei sich oberhalb und unterhalb der Bajonettverschlußverbindung (14) jeweils mindestens eine Dichtung (17, 18) befindet.

2. Elektrodenhalterung nach Anspruch 1, dadurch gekennzeichnet, daß die Dichtungen (17, 18) aus einer ersten unteren Ringdichtung (O-Ring 17, Quadring) für den Bajonettverschluß (14) und aus einer zweiten oberen Ringdichtung (O-Ring, Quadring) für das Elektrodensystem bestehen.

3. Elektrodenhalterung nach Anspruch 1 oder 2, dadurch gekennzeichnet, daß der Bajonettverschluß aus mindestens einer, vorzugsweise mehrerer, sich je nach Anzahl der aufzunehmenden Elektrodenmeßsysteme in peripherer Vorteilung am Außenumfang des inneren Lagerrohrs axial und radial erstreckenden Führungsnuten (15) besteht, in welche vom Innenumfang des äußeren Schutz- oder Halterohrs (12) ausgehend Vorsprünge (16a, 16b) eingreifen, oder umgekehrt.

4. Elektrodenhalterung nach einem der Ansprüche 1 bis 3, dadurch gekennzeichnet, daß zur Anschlagbildung zwischen den beiden Rohren (inneres Lagerrohr 11, äußeres Halterohr 12) eine Abschulterung zwischen beiden vorgesehen ist.

Fig.1

Fig.2

Europäisches
Patentamt

**EUROPÄISCHER
RECHERCHENBERICHT**

Nummer der Anmeldung

**EP 90 11 5117**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,A | US-A-4 578 170 (SHERIDAN M.)<br>* das ganze Dokument * | 1,2,3 | G 01 N 27/28 |
| A | PATENT ABSTRACTS OF JAPAN vol. 9, no. 269 (P-400)(1992) 26 Oktober 1985,<br>& JP-A-60 117144 (TOSHIBA K.K.) 24 Juni 1985,<br>* das ganze Dokument * | 1-3 | |
| A | PATENT ABSTRACTS OF JAPAN vol. 10, no. 41 (P-429)(2098) 18 Februar 1986,<br>& JP-A-60 186752 (OOKURA DENKI K.K.) 24 September 1985,<br>* das ganze Dokument * | 1,2,4 | |

| RECHERCHIERTE SACHGEBIETE (Int. Cl.5) |
|---|
| G 01 N |

Der vorliegende Recherchenbericht wurde für alle Patentansprüche erstellt

| Recherchenort | Abschlußdatum der Recherche | Prüfer |
|---|---|---|
| Den Haag | 07 Dezember 90 | BAROCCI S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument